# EUROPEAN PATENT SPECIFICATION

(11) **EP 2 004 845 B1**
(45) Date of publication and mention of the grant of the patent: **07.09.2011**
(21) Application number: 07732335.0
(22) Date of filing: 05.04.2007
(51) Int. Cl.: C12Q 1/37, G01N 33/558

(54) **A PROTEASE DETECTION PRODUCT**
PROTEASENACHWEISPRODUKT
PRODUIT DE DÉTECTION DE PROTÉASE

(30) Priority: 07.04.2006 GB 0607070
(43) Date of publication of application: 24.12.2008
(73) Proprietor: Mologic Ltd, Sharnbrook, Beds MK44 1LQ (GB)
(72) Inventor: DAVIS, Mark James, Souldrop, Bedfordshire MK44 1EZ (GB); DAVIS, Paul James, Felmersham, Bedfordshire MK43 7EX (GB); BURNAPP, Mark, Bedford, Bedfordshire MK41 9NB (GB)
(74) Representative: Spencer, Matthew Peter
(86) International application number: PCT/GB2007/001291
(87) International publication number: WO 2007/128980

(56) References cited:
- EP-A- 1 394 270
- WO-A-2006/006961
- US-A1- 2003 219 833

## Description

The present invention relates to a protease detection product which is suitable for detecting a protease enzyme in a sample.

Proteases are enzymes which digest proteins, in particular by hydrolyzing and cleaving peptide bonds. Thus a protein which is degraded by a protease is cleaved into two or more smaller peptides. Cleavage of a peptide occurs when proteases hydrolyze a peptide bond which is adjacent to a specific series of amino acid residues, although other proteases are less specific, and require only one or two amino acids to direct peptide cleavage activity:

An entirely separate area of development has been that of lateral flow immunoassays. These immunoassays allow the detection of analytes in samples. A common example of such an immunoassay is a pregnancy testing kit, which comprises a nitrocellulose strip on which are located, from one end to the other: an absorbent sample receiving area; a labelled antibody band; a capture antibody band and a control band.

The labelled antibody band comprises a plurality of monoclonal antibodies, specific for hCG (human chorionic gonadotropin) and conjugated to gold particles. The labelled antibodies are mobile in, and flow through, the nitrocellulose strip in the presence of a liquid. The capture antibody band comprises a plurality of immobilised monoclonal antibodies, each specific to hCG (although a different epitope thereof from the labelled antibodies). The control band comprises a plurality of immobilised antibodies specific for immunoglobulin G (IgG) from a particular species.

In order to use the kit to test for pregnancy in an individual, a sample (typically urine) from the individual is deposited on the sample receiving zone. The sample naturally flows (following capillary force) along the length of the strip towards firstly the labelled antibody band, then the capture antibody band and finally the control band. It is to be appreciated that the strip thus provides a liquid flow path for the sample.

As the contents of the sample pass the labelled antibody band, the labelled antibody is mobilised within the strip and is also carried in the direction of the capture antibody band. If hCG is present in the sample then the labelled antibody binds to the hCG.

When the labelled antibody reaches the capture antibody band, one of two things may happen. If hCG is present in the sample then the hCG is bound by the capture antibodies. The majority of the hCG will already be bound to the labelled antibodies and thus a complex is formed at the immobilised capture antibody, the hCG forming a link or bridge between the capture antibody and the labelled antibody. This is commonly known as an antibody "sandwich". Alternatively, if there is no hCG in the sample then the labelled antibody passes through the capture antibody band, without interacting with the capture antibodies. It is to be appreciated that the capture antibodies are immobilised and so in either case they do not flow along the strip.

Subsequently, the sample reaches the control band. Because there is an excess of labelled antibody in the kit, even if hCG is present in the sample, there is sufficient labelled antibody present in the kit to ensure some material passes beyond the capture antibody band. Thus, whether or not hCG is present in the sample, some labelled antibody reaches the control band. At the control band, the immobilised anti-IgG antibody binds to, and thus immobilises, the labelled antibody.

It is to be understood that where the labelled antibody is immobilised and concentrated, the presence of the gold particles forms a visible line. Thus, if hCG is present in the sample (which will be the case if the individual is pregnant) then a visible line forms at the capture antibody band. Irrespective of whether or not hCG is present in the sample, a visible line will form at the control band. The line at the control band is useful because it is indicative that the assay has reached its conclusion (which may not occur if, for example, there is insufficient liquid in the sample) and confirms the device has functioned correctly. It also permits a comparison of the capture antibody band with the control band in order to provide additional certainty to the result.

The present invention provides an improved product for detecting the presence of protease enzymes.

According to one aspect of the present invention, there is provided a protease detection product for detecting a protease enzyme in a sample comprising:
a medium providing a liquid flow path;
a cleavable component pre-immobilised on the liquid flow path at a first location, the cleavable component comprising a base element connected to a releasable element via a protease-sensitive linker peptide, the releasable element comprising a label binding structure and the base element being immobilized within the structure of the medium via an attachment between the base element and a structure on the liquid flow path ; a label capable of binding the label binding structure;
a capture component located downstream of the first location on the liquid flow path, the capture component being capable of binding the releasable element.

The cleavable component may be bound to the label (e.g. covalently) in which case the cleavable component may be regarded as a labelled component". Alternatively, the cleavable component may be a separate component from the label, for example, the label may comprise an antibody to the label binding structure on the releasable element of the cleavable component.

The cleavable component is pre-immobilised on the liquid flow path, in which case the "first location" is the position on the liquid flow path at which the cleavable component is located.

According to another aspect of the present invention, there is provided a protease detection product for detecting a protease enzyme in a sample comprising:
a medium providing a liquid flow path;
a labelled component pre-immobilised on the liquid flow path, the cleavable component comprising a base element connected to release of an element via a protease-sensitive link of peptide. The releasable element comprising a label, and the base element being immobilized within the structure of the medium via an attachment between the base element and a structure on the liquid flow path ;
a capture component located downstream of the label component. On the liquid flow path, the capture component being capable of binding the releasable element.

Preferably, the medium providing the liquid flow path is an absorbent strip, for example, one made from nitrocellulose.

Conveniently, the immobilisation means comprises an attachment between the base element and a structure on the liquid flow path. For example the base element may comprise biotin and the structure on the liquid flow path is streptavidin.

Conveniently, the protease detection product further comprises a protease inhibitor located upstream of the capture component or capture components on the liquid flow path.

Preferably, the protease detection product further comprises a degradable or disruptable barrier on the liquid flow path. The barrier may be between 10 and 100 µm thick. For example, in some embodiments, the barrier is a soluble barrier such as collagen, pectin or PVA.

Conveniently, the degradable or disruptable barrier comprises a lip, extending outwardly of the liquid flow path, for preventing material passing around the barrier when passing along the liquid flow path. The lip may extend outwardly for at least 0.5 mm. Genereally the lip extends outwardly for less than 5 mm, preferably less than 2mm. A lip of around 1 mm is most preferred.

Advantageously, the protease detection product further comprises a sample receiving zone for the mixing of the cleavable component and the sample, wherein the first location is within the sample receiving zone and wherein the degradable or disruptable barrier being located downstream of the sample receiving zone and upstream of the capture component or components.

Preferably, the or each capture component comprises one half of a binding pair.

Conveniently, the or each capture component comprises an antibody or an antigen binding fragment thereof.

Alternatively, the or each capture component comprises cellulose, the releasable element further comprising a binding moiety capable of being bound by cellulose.

Preferably, the binding moiety is galactomannan or xyloglucan.

Advantageously, label is a gold particle or a fluorophore group.

Alternatively the or each capture component comprises one half of a biotin-streptavidin binding pair. Preferably, the protease detection product is a lateral flow immunoassay.

According to another aspect of the present invention, there is provided a method for detecting a protease enzyme in a sample using a protease detection product according to the invention comprising the steps of: providing the sample at the upstream end of the liquid flow path and allowing the sample to pass along the liquid flow path; and determining the presence of the label at the capture component and/or at the first location.

Conveniently, the method further comprises the step of separately adding the label to the upstream end of the liquid flow path and allowing it to pass along the liquid flow path.

Preferably, the method further comprises the step of applying a wash buffer to the upstream end of the liquid flow path after the step of applying the sample to the liquid flow path. For example, between 50 and 500 µl of wash buffer may be added; around 150 µl being most preferred.

In this specification, where reference is made to a component being "on" a liquid flow path, it may equally be "in" the liquid flow path and vice versa.

In order that the present invention may be more fully understood and so that further features thereof may be appreciated, embodiments of the invention will now be described, by way of example, with reference to the accompanying drawings in which:
Figure 1 is a plan view of a protease detection product in accordance with one embodiment of the present invention;
Figure 2 is a schematic view of one component of the protease detection product of Figure 1;
Figure 3 is a schematic view of another component of the protease detection product shown in Figure 1 while in use;
Figure 4 is a perspective view of a protease detection product in accordance with a further embodiment of the present invention;
Figure 5 is a plan view of a protease detection product in accordance with another embodiment of the present invention; and
Figure 6 is a schematic longitudinal cross-sectional view of a protease detection product tested in Example 3.

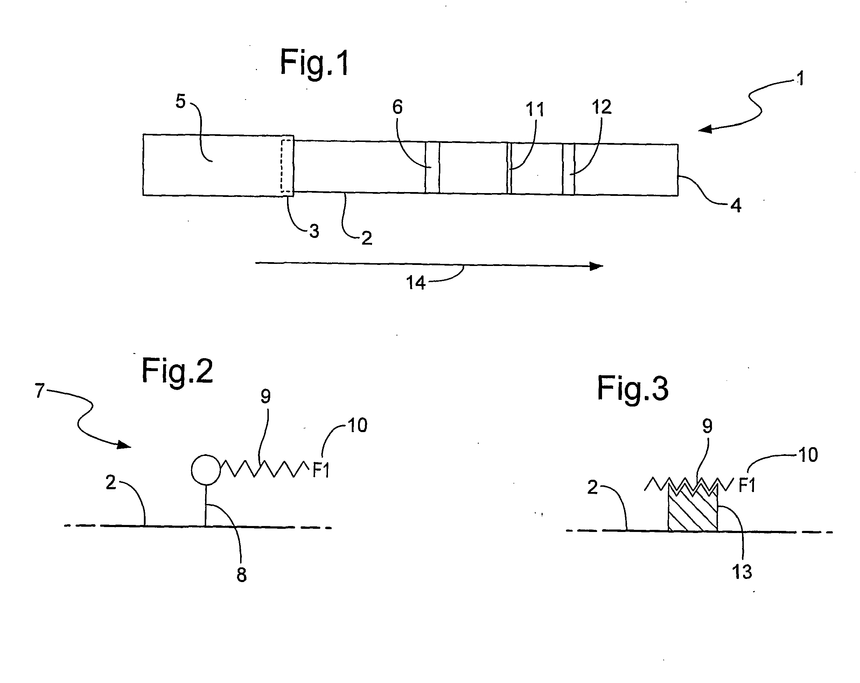

Referring to Figure 1, a protease detection product 1 comprises a nitrocellulose strip 2 having first and second ends 3, 4. Attached to the first end 3 is a sample receiving zone 5 which comprises an elongate, rectangular absorbent material of slightly greater width than the nitrocellulose strip 2.

Further towards the second end 4 is provided a labelling band 6 which is arranged transverse to the long axis of the strip 2. The labelling band 6 comprises a plurality of labelled components 7 which are shown in further detail in Figure 2. The labelled component 7 comprises a stem 8 which is immobilised within the strip 2 structure to enhance the access of the enzyme to the labelled component 7. Attached to the stem 8 is provided a protease-sensitive linker peptide 9. Attached to the free end of the protease-sensitive linker peptide 9 is provided a label 10, which in this case is a fluorophore but in other embodiments is, for example, a gold particle or a chromogenic moiety.

Further along the strip 2, towards the second end 4, is provided a protease inhibitor band 11, which is located transverse to the long axis of the strip 2. The protease inhibitor band 11 comprises a protease inhibitor dried into the strip 2. It is to be understood that the protease inhibitor band 11 is by no means essential to the invention and is omitted in some other embodiments.

Further along the strip 2 towards the second end 4 is provided a capture band 12, which is also located transverse to the long axis of the strip 2. The capture band 12 comprises a plurality of capture components 13, one of which is shown schematically in Figure 3. In this embodiment, the capture molecule 13 is a monoclonal antibody, immobilised into the strip 2 but this is not essential to the invention. What is important is that each capture component 13 is capable of binding the free end of the peptide linker 9. In Figure 3, the capture component 13 is illustrated binding the peptide linker 9 but it is to be understood that this illustrates the protease detection product in use. Before the protease detection product 1 is used, no components are bound to the capture component 13.

In order to detect an active protease enzyme in a liquid sample using the protease detection product 1, the sample is deposited on the sample receiving zone 5 and subsequently absorbed into the nitrocellulose strip 2, adjacent the first end 3. A chase buffer comprising a suitable solvent is then deposited on the sample receiving zone in order to ensure that there is sufficient liquid to carry the sample along the strip 2. The sample is then absorbed towards the second end 4 of the strip 2, in the direction of the arrow 14 along what is referred to as the liquid flow path.

When the sample reaches the labelling band 6, any protease enzyme in the sample cleaves the protease-sensitive linker peptide 9 and thus releases the free end of the linker peptide together with the label 10. If, on the other hand there is no active protease in the sample then the linker peptide 9 remains intact and is thus immobilised to the strip 2 via the stem 8.

The sample then continues along the liquid flow path until it reaches the protease inhibitor band 11 at which point any active protease enzyme in the sample is deactivated.

The sample continues along the liquid flow path until it reaches the capture band 12 and the plurality of capture components 13. Since any active protease enzyme in the sample has been deactivated, the sample does not degrade the capture component 13 itself.

If an active protease was in the original sample and has thus cleaved the protease-sensitive linker peptide 9 then the free end of the linker peptide 9 binds to the capture component 13 and remains immobilised on the strip 2. If, on the other hand, there was no active protease in the original sample or any protease in the sample was not capable of cleaving the specific linker peptide 9 then the capture component 13 has nothing to which to bind.

Subsequently, the capture band 12 is visualised. If the label is a fluorophore, as in the present embodiment, then this is carried out under ultraviolet light but if the label is visible under natural light (for example if the label is a gold particle) then ultraviolet light is unnecessary. If the peptide linker 9 has been cleaved and is thus bound to the capture component 13 then the accumulation of the label 10 at the capture band 12 is visible as a line on the strip 2. If, on the other hand, the sample did not contain a protease to which the peptide linker 9 was sensitive then there is no accumulation of the label 10 at the capture band 12. Thus the presence of an active protease enzyme in the sample can be assessed by observing the presence or absence of a line at the capture band 12, once the assay has completed.

It is to be appreciated that if no protease is present in the sample then the label 10 remains at the labelling band 6. Thus in some alternative embodiments, instead of determining the presence of a line of the label at the capture band 12, the presence of a line at the labelling band 6 is measured. If the label is present at the labelling band 6 then it is indicative of the absence of protease in the sample but if the label is not present at the labelling band 6 or is present at a reduced intensity then it is indicative of the presence of a protease in the sample.

In further embodiments of the present invention, the quantity of the label at the labelling band 6 and the capture band 12 (in other words the intensity of the line of the respective bands 6, 12) are both determined in order assess the ratio of the label at the two bands. By determining the ratio of the label at the two bands, it is possible to quantify the amount of, or activity of, the protease enzyme in the sample. More specifically, the greater the amount of label at the capture band 12 compared with the labelling band 6, the more protease enzyme, or the more active the protease enzyme is in the sample and *vice versa*.

In the above-described embodiment of the invention, the capture component is an antibody. However, in further embodiments of the present invention, the capture components are components other than antibodies. For example, in some embodiments, the protease-sensitive peptide contains a carbohydrate moiety (displaying a CIS diol) and the capture component is phenylboronic acid, which is capable of binding the carbohydrate. In one particularly preferred embodiment, the capture component 13 is cellulose and the protease-sensitive linker peptide 9 is conjugated to galactomannan or xyloglucan, either of which will bind to cellulose. The advantage of such embodiments is that the protease inhibitor band 11 is not required because cellulose, galactomannan and xyloglucan are not affected by protease enzymes. Other examples of binding pairs that may be used are lectin (e.g. lectin from elderberry) with a carbohydrate (a sugar O-linked to the peptide); and streptavidin with biotin

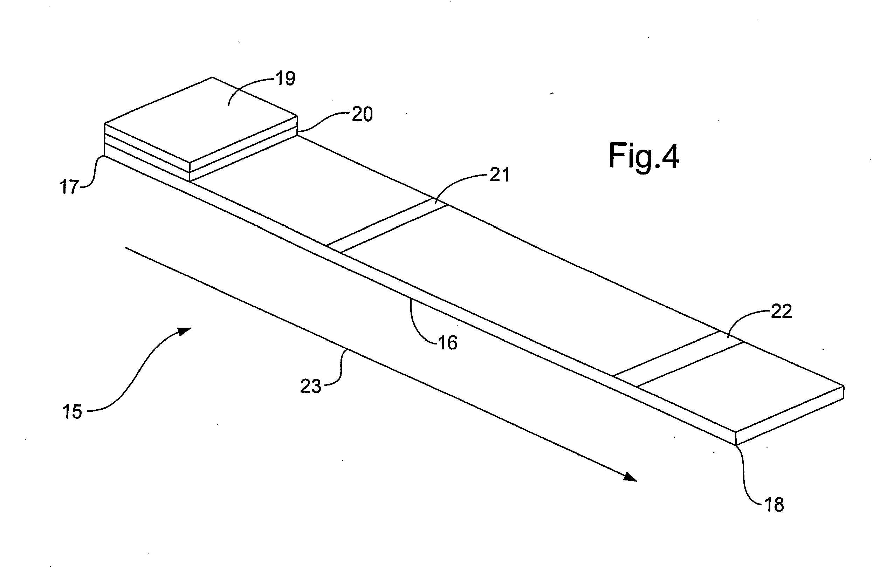

Referring now to Figure 4, a second embodiment of the present invention is shown. A protease detection product 15 comprises a nitrocellulose strip 16 having first and second ends 17, 18. At the first end of the nitrocellulose strip 16 is located a sample receiving zone 19 which comprises a rectangular sheet of an absorbent material which sits above the strip 16. Impregnated in the sample receiving zone 19 is a protease-sensitive peptide conjugated to a label such as a fluorophore group. The protease sensitive peptide has a cleavage sequence at which the protease can cleave the peptide. Sandwiched between the nitrocellulose strip 16 and the sample receiving zone 19 is a soluble barrier 20 made from, for example, pectin or polyvinyl alcohol. The soluble barrier 20 is temporarily impermeable to liquid and dissolves in the presence of water and thus becomes permeable after a predetermined period of time.

Further along the nitrocellulose strip 16, towards the second end 18 is provided a first capture band 21 which is located transverse to the long axis of the strip 16. The first capture band 21 comprises a plurality of monoclonal antibodies, each immobilised on the surface of the strip 16. The antibodies in the first capture band 21 are specific for a binding sequence of the peptide located such that the cleavage sequence is between the binding sequence and the label. In some alternative embodiments, the antibodies of the first capture band 21 are specific for the cleavage sequence itself or are specific for a conformational epitope that is disrupted through the action of the protease enzyme.

Further along the strip 16 towards the second end 18 thereof is provided a second capture band 22, which is also located transverse to the long axis of the strip 16. The second capture band comprises a plurality of monoclonal antibodies, each immobilised into the surface of the strip 16. The antibodies in the second capture band 22 are specific for a sequence on the peptide between the cleavage sequence and the label.

In order to use the protease detection product 15 to detect the presence of an active protease enzyme in an aqueous sample, the sample is deposited onto the sample receiving zone 19. A chase buffer, as described in relation to the previous embodiment, is also deposited on the sample receiving zone 19. The sample soaks through the sample receiving zone 19 but is initially incapable of being absorbed further because of the presence of the soluble barrier 20. After a period of time, the buffer in the sample dissolves the soluble barrier 20. During that period of time, however, the sample thoroughly mixes with the protease-sensitive peptide therein and any protease present in the sample will cleave the peptide at the cleavage sequence.

Once the soluble barrier 20 becomes permeable, the sample, mixed with the protease-sensitive peptide, is absorbed along the nitrocellulose strip 16 in the direction of the arrow 23 along a liquid flow path.

The mixture of sample and peptide then passes through the first capture band 21. If there was any active protease enzyme in the sample capable of cleaving the peptide then the peptide will have been cleaved and the antibody will bind a peptide fragment that does not carry the label. In the alternative embodiments, no peptide will be able to bind to the antibody in the first capture band because the antibody is specific for the sequence across the cleaving point, or is specific for a conformational epitope that is disrupted through the action of the protease enzyme activity. If, on the other hand, there was no active protease in the sample capable of cleaving the peptide then intact peptide will bind to the antibodies in the first capture band 21.

The mixture of peptide and sample then continues along the strip 16 in the direction of the arrow 23 where it reaches the second capture band 22. Whether or not protease was present in the sample, the intact or cleaved peptide will bind to the antibodies in the second capture band 22. It is to be appreciated that if the protease is in the sample then little or no label will become bound to the first capture band 21 so more label is available to be bound to the second capture band 22.

The strip 16 is then visualised in order to locate the label on it. If the label is a fluorophore group as in this embodiment, then the strip 16 is visualised under ultraviolet light. Where the label has accumulated, it is visible as a line on the strip 16. If the label has accumulated at the first capture band 21 then this is indicative of the absence of an active protease enzyme in the sample.

In an alternative embodiment, the presence of the label at the second capture band 22 is determined, the presence of label at the second capture band 22 being indicative of the presence of an active protease enzyme in the sample.

In some further embodiments, the presence of label is determined at both the first and second capture bands 21, 22 and the ratio of the intensity of the lines of label at the two bands 21, 22 is assessed. The more label there is at the second capture band 22 as compared with the first capture band 21 being indicative of the more protease enzyme, or a more active enzyme, in the sample and *vice versa*.

It is also to be understood that the second capture band 22 acts as a "control band" because label accumulation at the second capture band 22 indicates that the sample has penetrated the soluble barrier 20 and has reached the end of the assay.

In some alternative embodiments, the location of the first and second capture bands, 21, 22 is reversed, i.e. the second capture band 22 is upstream of the first capture band.

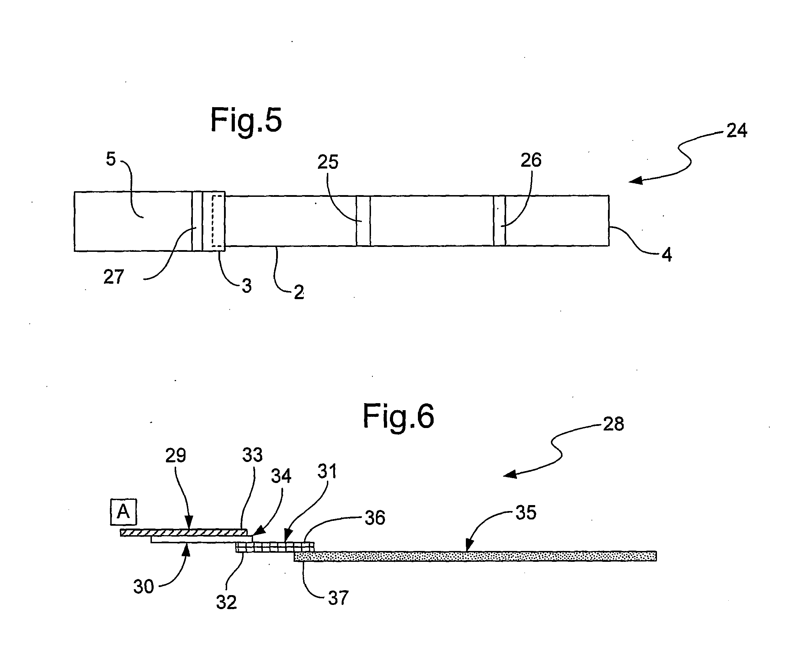

It is to be appreciated that in the embodiment shown in Figure 5, the advantage of providing the soluble barrier 20 is that thorough mixing of the protease-sensitive peptide and the sample can occur before the mixture proceeds along the liquid flow path. If the soluble barrier 20 were not provided then the nitrocellulose strip 16 would have to be much longer in order to provide sufficient time for the mixing to occur and the protease to cleave the peptide before the mixture reached the first capture band 21.

Referring, now, to Figure 5, a variant of the embodiment shown in Figure 1 is depicted with like components give the same reference numerals. In this embodiment, a protease detection product 24 differs from the product shown in Figure 1 in that the labelling band 6 and the capture band 12 are replaced with a capture band 25 and a further capture band 26, respectively. Furthermore, the protease inhibitor band 11 is omitted and a soluble barrier 27 is provided in the sample receiving zone 5, separating the main part of the sample receiving zone 5 from the first end 3 of the nitrocellulose strip 2.

Dried into the main part of the sample receiving zone 5 is a protease-sensitive peptide conjugated to a label such as a fluorophore group. The protease-sensitive peptide has a cleavage sequence at which a protease can cleave the peptide.

The capture band 25 comprises a plurality of monoclonal antibodies, each immobilised onto the surface of the strip 2. The antibodies in the capture band 25 are specific for a sequence on the peptide between the cleavage sequence and the label.

The further capture band 26 comprises a plurality of monoclonal antibodies, each immobilised on the surface of the strip 2. The antibodies in the further capture band 26 are specific for a binding sequence of the peptide located such that the cleavage sequence is between the binding sequence and the label. In some alternative embodiments, the antibodies of the further capture band 26 are specific for the cleavage sequence itself or are specific for a conformational epitope which is disrupted through the action of the protease enzyme.

The protease detection product 24, is used as in the previous embodiments. Thus a sample is deposited on the sample receiving zone 5 and a chase buffer is added. The sample then mixes with the protease sensitive peptide on the sample receiving zone 25 while the soluble barrier 27 is dissolved by the buffer. If there is a suitable protease in the sample then the protease-sensitive peptide is cleaved.

After a period of time, the soluble barrier 27 is degraded and the mixture of sample, buffer and protease-sensitive peptide passes along the strip 2 from the first end 3 to the second end 4. As the mixture passes the capture band 25, the antibodies in the capture band 25 bind to the protease-sensitive peptides, irrespective of whether or not it has been cleaved by a protease. However, because there is an excess of the protease-sensitive peptide provided in the sample receiving zone 5, some of the protease-sensitive peptide passes beyond the capture band 25 and reaches the further capture band 26.

At the further capture band 26, the antibodies bind and immobilise the binding sequence of the peptide. If the protease-sensitive peptide has been cleaved then the label is no longer attached to the binding sequence and so the label is not immobilised at the further capture band 26. In the alternative embodiments, the peptide does not bind to the antibodies of the further capture band 26, at all, if there has been cleavage of the protease-sensitive peptide and so for this reason the label does not accumulate at the further capture band 26. If, on the other hand, the protease-sensitive peptide has not been cleaved then the label does accumulate at the further capture band 26.

Once the sample has passed the further capture band 26, the strip 2 is inspected to determine the outcome of the assay. The level of label which accumulates at the further capture band 26 is compared with the level of label accumulated at the capture band 25. The lower the level of label at the further capture band 26, the greater the amount, or the greater the activity of the protease in the sample (and *vice versa*) since it is indicative of a greater amount of cleavage of the protease-sensitive peptide.

It is to be appreciated that in the other versions of this embodiment, the position of the capture band and the further capture band 26 may be reversed with respect to each other (i.e. the further capture band 26 may be located upstream on the liquid flow path of the capture band 25).

In one particular version of this embodiment, the label comprises a gold particle to which is conjugated the protease-sensitive peptide. Also conjugated to the gold particle is a galactomannan moiety. The capture band 25 comprises a plurality of antibodies capable of binding a binding sequence on the peptide, the cleavage sequence being between the gold particle and the binding sequence. The further capture band 26 comprises PBA, which binds carbohydrate displaying a CIS diol. In the absence of protease, gold particles are bound at and accumulate at both the capture band 25 and the further capture band 26. In the presence of a suitable protease, however, the cleavage sequence is cleaved and so any peptide bound at the first capture band 25 is not attached to a gold particle. Thus gold particles only accumulate at the further capture band 26 in the presence of the protease. Accordingly, the presence or absence of an active protease enzyme in the sample can be assessed by comparing the accumulation of the gold particle at the capture band 25 with gold particle accumulation at the further capture band 26.

### EXAMPLES

### Example 1:- The Cleavage of Immobilised Peptide on the Strip.

Using an immunoassay test strip containing a streptavidin test line 1 and an anti-mouse antibody test line 2 the following assay was performed to show the protease cleavage of an immobilised peptide. A cleavable peptide (Fluorescein-Val-Arg-Gly-[PEG]₂₀-Biotin (a)) was immobilised to the strip via streptavidin / biotin binding and subsequently cleaved by running Trypsin containing solution (which is capable of cleaning the peptide at the C-terminal bond of the Arg residue) up the strip:
a) 4µl of 0.65µg/ml peptide (Fluorescein-Val-Arg-Gly-[PEG]₂₀-Biotin) was mixed with 10µl of Bovine Serum Albumin (200mg/ml BSA) and 20µl 0.01 M phosphate buffered saline pH 7.4 (PBS) and added to the base of the 6 strips held vertically on a support and allowed to chromatograph up each strip.
b) When all mixture prepared in (a) had soaked into each strip a 150µl PBS wash was added to the base of each strip and allowed to chromatograph up each strip.
c) When the wash in (b) had been drawn through each strip the strips were allowed to dry for 30 minutes at room temperature.
d) Following drying 20µl volumes of Trypsin standards of 0, 50, 100, 250, 500 and 1000µg/ml were added to the base of individual strips and allowed to chromatograph up each strip.
e) When the standard added in (d) had been drawn through each strip, a further 50µl PBS wash was added to the base of each strip and allowed to chromatograph up each strip.
f) When the wash in (e) had been drawn into each strip 2µl of anti-FITC gold conjugate and 100µl PBS mixed and added to the base of each strip and allowed to chromatograph up each strip followed by a further 80µl PBS wash per strip. The anti-FITC gold conjugate is capable of binding Fluorescein and the conjugate can be bound and immobilised by the anti-mouse antibody at test line 2.

The results of signals strengths for test lines 1 and 2 are tabulated in Table 1 below. It is to be noted that the presence of the gold conjugate at either test line not directly detected. Fluorescein was used in the cleavable peptide not for its fluorescent properties but because it is a small, antigenic molecule and several antibodies against it are commercially available.

**Table 1 - Signal Generation for test lines 1 and 2 using Peptide Immobilised on Strip**

| Trypsin Standard | Test line 1 | Test Line 2 |
|---|---|---|
| 0µg/ml | ++++ | + |
| 50µg/ml | +++ | ++ |
| 100µg/ml | ++ | +++ |
| 250µg/ml | ++ | +++ |
| 500µg/ml | + | +++ |
| 1000µg/ml | + | +++ |

### Example 2:- The use of Fluorescent Label for Visualisation of Result.

Using an immunoassay test strip containing a streptavidin test line 1 and an anti-mouse antibody test line 2 the following assay was performed with a cleavable peptide (Fluorescein-Val-Arg-Gly-[PEG]₂₀-Biotin) using fluorescence for the detection of Trypsin activity in the sample:
a) 4µl volumes of 50µg/ml peptide (Fluorescein-Val-Arg-Gly-[PEG]₂₀-Biotin) and mixed with 4µl volumes of Trypsin standard (0, 10, 100 and 1000µg/ml) and incubated for 5 minutes at Room Temperature.
b) To mixture prepared in (a), 82µl of 33mg/ml BSA in PBS was added, further mixed and then added to the base of the 4 strips held vertically on a support and allowed to chromatograph up each strip.
c) When all mixture prepared in (b) had been drawn into each strip a 150µl PBS wash was added to the base of each strip and allowed to chromatograph up each strip.
d) Strips were viewed using a UV light source to visualise presence of uncleaved peptide bound to test line 1.

The results of signals strengths for test line 1 are tabulated in Table 2 below. In this example the presence of Fluorescein generates the signal.

**Table 2 - Signal Generation for test line 1 using fluorescent label for visualisation.**

| Trypsin Standard | Test line 1 |
|---|---|
| 0µg/ml | +++ |
| 10µg/ml | + |
| 100µg/ml | - |
| 1000µg/ml | - |

### Example 3:- The incorporation of the cleavable peptide into the sample pad of a test strip.

Using an immunoassay test strip containing a streptavidin test line 1 and an anti-mouse antibody test line 2 the following assay was performed to show Trypsin cleavage of a cleavable peptide (Fluorescein-Val-Arg-Gly-[PEG]₂₀-Biotin) supplied dried in the sample pad of the test strip:
a) The following solution prepared and then soaked into a 60*32mm length sample pad and dried for 3 hours at Room Temperature:
   1ml of 200mg/ml BSA + 500µl of 1% Triton X-100 + 2ml d.H₂O + 2g Sucrose + 500µl of 50µg/ml cleavable peptide.
b) Strips were fabricated as shown in Figure 6 and as described in greater detail below. In brief, a sample receiving pad 29 contains dried cleavable peptide. Separating the sample receiving pad 29 from an intermediate pad 31 is a dissolvable PVA film 30. The intermediate pad 31 contains anti-FITC gold conjugate and is in contact with an absorbent test trip 35 which contains test lines 1 and 2.
c) Assays were run using Trypsin standards of 0 and 1000µg/ml by applying standard to the proximal end of the sample pad labelled 'A' until this pad was completely saturated.
d) When the PVA film between the sample pad and conjugate was seen to dissolve allowing standard to pass into the conjugate pad further solution was applied as required to allow the strip to run to completion.

The results of signals strengths for test lines 1 and 2 are tabulated in Table 3 below.

**Table 3 - Signal Generation for test lines 1 and 2 using cleavable peptide incorporated into sample pad of test strip.**

| Trypsin Standard | Test line 1 | Test Line 2 |
|---|---|---|
| 0µg/ml | +++ | ++ |
| 1000µg/ml | - | +++ |

Referring, now, to Figure 6, the immunoassay test strip 28 comprises, at a first end, a sample receiving pad 29 made from an absorbent material. A dried, cleavable peptide (Fluorescein-Val-Arg-Gly-[PEG]₂₀-Biotin) is dried onto the sample receiving pad 29.

Located beneath the sample receiving pad 29 is a PVA film 30, of 35µm thickness. The PVA film 30 is sized so as to cover the entire inner end of the sample receiving pad 29 and extend an additional 1 mm around all dimensions of the sample receiving pad 29 (except on the outer end of the sample receiving pad 29). The PVA film 30 is a barrier to fluid passing beyond the sample receiving pad 29 but is soluble in water after a predetermined length of time.

Located beneath the PVA film 30 is an intermediate pad 31. The intermediate pad 31 is located so that the outer end thereof 32 overlaps with the inner end 33 of the sample receiving pad 29 with the inner end 34 of the PVA film 30 being sandwiched therebetween. Furthermore, the 1 mm extension of the PVA film 30 from the inner end 33 of the sample receiving pad 29 prevents any direct fluid communication between the sample receiving pad 29 and the intermediate pad 31. A plurality of anti-FITC gold conjugates are dried onto the intermediate pad 31.

Located beneath the intermediate pad 31 is an absorbent strip 35. The inner end 36 of the intermediate pad 31 overlaps with, and is in contact with the outer end 37 of the absorbent strip 35. Located on the absorbent strip 35 are first and second test lines (not shown). The first test line, which is closest to the intermediate pad 31 comprises streptavidin immobilised on the surface of the absorbent strip 35. The second test line, which is remote from the intermediate pad 31 comprises a plurality of anti-mouse antibodies immobilised on the absorbent strip 35.

In use, a protease-containing sample is added to the proximal end of the sample receiving pad 29, at the point marked A. The sample mixes with the cleavable peptide located in the sample receiving pad 29 and cleaves the peptide. Furthermore, the sample dissolves the PVA film 30 and, after a predetermined length of time, the cleaved peptide is capable of flowing to the intermediate pad 31 where it mixes with the anti-FITC gold conjugate. The mixture of cleaved peptide and anti-FITC gold conjugate is then absorbed onto the absorbent strip 35 where the biotin containing section of the cleaved peptide binds at the first test line and the Fluorescein-containing part of the cleaved peptide, binds to the anti-FITC gold conjugate which is immobilised at the second test line.

Alternatively, in the absence of a protease in the sample, the peptide remains uncleaved on the sample receiving pad 29; the sample dissolves and passes through the PVA film 30 and mixes with the FITC gold conjugate on the intermediate pad 31. The anti-FITC gold conjugate binds to the Fluorescein of the uncleaved peptide, which is then immobilised at the first test line. A small amount of the anti-FITC gold conjugate, in practice, does not bind to the cleavable peptide and the unbound conjugate is immobilised at the second test line.

It is to be appreciated that, in practice, not all of the cleavable peptide is cleaved even when the sample contains a protease and thus the relative amounts of the anti-FITC gold conjugate immobilised at the first and second test lines is indicative of the amount and/or efficacy of the protease in cleaving the cleavable peptide.

It is also to be understood that the anti-FITC gold conjugate is located in the intermediate pad 31 rather than the sample receiving pad 29 so as to minimise the exposure of the anti-FITC conjugate to the protease in a sample, which could otherwise exercise its proteolytic activity against the conjugate. Accordingly, by locating the anti-FITC gold conjugate in the intermediate pad 31, any proteolysis of the conjugate is minimised and thus the signal produced by the gold conjugate at the test lines is maximised.

## Claims

1. A protease detection product for detecting a protease enzyme in a sample comprising:
a medium providing a liquid flow path;
a cleavable component pre-immobilised on the liquid flow path at a first location, the cleavable component comprising a base element connected to a releasable element via a protease-sensitive linker peptide, the releasable element comprising a label binding structure and the base element being immobilised within the structure of the medium via an attachment between the base element and a structure on the liquid flow path;
a label capable of binding the label binding structure; and
a capture component located downstream of the first location on the liquid flow path, the capture component being capable of binding the releasable element.

2. A protease detection product according to claim 1 further comprising a protease inhibitor located upstream of the capture component on the liquid flow path.

3. A protease detection product according to claim 1 or 2 further comprising a degradable or disruptable barrier on the liquid flow path.

4. A protease detection product according to Claim 3, wherein the degradable or disruptable barrier comprises a lip, extending outwardly of the liquid flow path, for preventing material passing around the degradable or disreputable barrier when passing along the liquid flow path.

5. A protease detection product according to claim 3 or 4 further comprising a sample receiving zone for the mixing of the cleavable component and the sample, wherein the first location is within the sample receiving zone and wherein the degradable or disruptable barrier is located downstream of the sample receiving zone and upstream of the capture component.

6. A protease detection product according to any one of the preceding claims wherein the capture component comprises one half of a binding pair.

7. A protease detection product according to claim 6 wherein the capture component comprises an antibody or an antigen binding fragment thereof.

8. A protease detection product according to claim 6 wherein the capture component comprises cellulose, the releasable element further comprising a binding moiety capable of being bound by cellulose.

9. A protease detection product according to claim 8 wherein the binding moiety is galactomannan or xyloglucan.

10. A protease detection product according to claim 6 wherein the capture component comprises biotin or streptavidin.

11. A protease detection product according to any one of the preceding claims wherein the label is a gold particle or a fluorophore group.

12. A method for detecting a protease enzyme in a sample using a protease detection product according to any one of the preceding claims comprising the steps of: providing the sample at the upstream end of the liquid flow path and allowing the sample to pass along the liquid flow path; and determining the presence of the label at the capture component and/or at the first location.

13. A method according to claim 12 further comprising the step of separately adding the label to the upstream end of the liquid flow path and allowing it to pass along the liquid flow path.

14. A method according to claim 12 or 13 further comprising the step of applying a wash buffer to the upstream end of the liquid flow path after the step of applying the sample to the liquid flow path.

## Patentansprüche

1. Protease-Nachweisprodukt zum Nachweis eines Protease-Enzyms in einer Probe, welches Folgendes aufweist:
ein Medium, welches einen Flüssigkeitsdurchflussweg bereitstellt;
eine spaltbare Komponente, welche an einer ersten Stelle auf dem Flüssigkeits-Durchflussweg vorimmobilisiert wird, wobei die spaltbare Komponente ein Basiselement aufweist, welches mit einem lösbaren bzw. abtrennbaren Element über ein protease-empfindliches Linker-Peptid verbunden ist, wobei das lösbare Element eine Markerbindestruktur aufweist und das Basiselement in der Struktur des Mediums über eine Bindung zwischen dem Basiselement und einer Struktur auf dem Flüssigkeitsdurchflussweg immobilisiert ist;
einen Marker, welcher in der Lage ist, die Markerbindestruktur zu binden; und
eine Einfangkomponente, welche stromabwärts der ersten Stelle auf dem Flüssigkeitsdurchflussweg angeordnet ist, wobei die Einfangkomponente in der Lage ist, das lösbare bzw. abtrennbare Element zu binden.

2. Protease-Nachweisprodukt nach Anspruch 1, welches des Weiteren einen Protease-Inhibitor bzw. -Hemmstoff aufweist, welcher stromaufwärts der Einfangkomponente auf dem Flüssigkeitsdurchflussweg angeordnet ist.

3. Protease-Nachweisprodukt nach Anspruch 1 oder 2, welches des Weiteren eine abbaubare oder trennbare Sperre auf dem Flüssigkeitsdurchflussweg aufweist.

4. Protease-Nachweisprodukt nach Anspruch 3, **dadurch gekennzeichnet, dass** die abbaubare oder trennbare Sperre eine Lippe aufweist, welche sich von dem Flüssigkeitsdurchflussweg nach außen erstreckt, um zu verhindern, dass Masse um die abbaubare oder trennbare Sperre herumgeführt wird, wenn sie entlang des Flüssigkeitsdurchflusswegs läuft.

5. Protease-Nachweisprodukt nach Anspruch 3 oder 4, welches des Weiteren eine Probe-Aufnahmezone zum Mischen der spaltbaren Komponente mit der Probe aufweist, **dadurch gekennzeichnet, dass** die erste Stelle innerhalb der Probe-Aufnahmezone liegt und dass die abbaubare oder trennbare Sperre stromabwärts der Probe-Aufnahmezone und stromaufwärts der Einfangkomponente angeordnet ist.

6. Protease-Nachweisprodukt nach einem der vorhergehenden Ansprüche, **dadurch gekennzeichnet, dass** die Einfangkomponente eine Hälfte eines Bindungspaares aufweist.

7. Protease-Nachweisprodukt nach Anspruch 6, **dadurch gekennzeichnet, dass** die Einfangkomponente einen Antikörper oder ein Antigen bindendes Antikörperfregment aufweist.

8. Protease-Nachweisprodukt nach Anspruch 6, **dadurch gekennzeichnet, dass** die Einfangkomponente Zellulose aufweist, wobei das abtrennbare bzw. lösbare Element des Weiteren einen Bindungsteil aufweist, welcher durch Zellulose gebunden werden kann.

9. Protease-Nachweisprodukt nach Anspruch 8, **dadurch gekennzeichnet, dass** der Bindungsteil Galaktomannan oder Xyloglucan ist.

10. Protease-Nachweisprodukt nach Anspruch 6, **dadurch gekennzeichnet, dass** die Einfangkomponente Biotin oder Streptavidin aufweist.

11. Protease-Nachweisprodukt nach einem der vorhergehenden Ansprüche, **dadurch gekennzeichnet, dass** die Markierung ein Goldpartikel oder eine fluorophore Gruppe ist.

12. Verfahren zum Nachweisen eines Protease-Enzyms in einer Probe, welches ein Protease-Nachweisprodukt nach einem der vorhergehenden Ansprüche verwendet, wobei das Verfahren die folgenden Schritte aufweist:
Bereitstellen der Probe an dem stromaufwärts gelegenen Ende des Flüssigkeitsdurchflusswegs und Zulassen, dass die Probe entlang des Flüssigkeitsdurchflusswegs läuft; und Bestimmen des Vorhandenseins des Markers an der Einfangkomponente und/oder an der ersten Stelle.

13. Verfahren nach Anspruch 12, welches des Weiteren den Schritt der separaten Hinzufügung des Markers an dem stromaufwärts gelegenen Ende des Flüssigkeitsdurchflusswegs aufweist, und Zulassen, dass er entlang des Flüssigkeitsdurchflusswegs läuft.

14. Verfahren nach Anspruch 12 oder 13, welches des Weiteren den Schritt des Anbringens eines Waschpuffers an dem stromaufwärts gelegenen Ende des Flüssigkeitsdurchflusswegs nach dem Schritt der Anwendung der Probe bei dem Flüssigkeitsdurchflussweg aufweist.

## Revendications

1. Produit de détection de protéase pour détecter une enzyme de protéase dans un échantillon, comprenant:
un milieu réalisant un chemin d'écoulement de liquide;
un composant clivable pré-immobilisé sur le chemin d'écoulement de liquide à un premier emplacement, le composant clivable comprenant un élément de base relié à un élément libérable par un peptide de liaison réagissant à la protéase, l'élément libérable comprenant une structure de liaison de marqueur, et l'élément de base étant immobilisé dans la structure du milieu par un attachement entre l'élément de base et une structure sur le chemin d'écoulement de liquide;
un marqueur apte à se lier à la structure de liaison de marqueur; et
un composant de capture situé en aval du premier emplacement sur le chemin d'écoulement de liquide, le composant de capture étant apte à se lier à l'élément libérable.

2. Produit de détection de protéase selon la revendication 1, comprenant en outre un inhibiteur de protéase situé en amont du composant de capture sur le chemin d'écoulement de liquide.

3. Produit de détection de protéase selon la revendication 1 ou 2, comprenant en outre une barrière dégradable ou apte à être rompue sur le chemin d'écoulement de liquide.

4. Produit de détection de protéase selon la revendication 3, dans lequel la barrière dégradable ou apte à être rompue comprend une lèvre, s'étendant vers l'extérieur du chemin d'écoulement de liquide, pour empêcher qu'un matériau passe autour de la barrière dégradable ou apte à être rompue lorsqu'il passe le long du chemin d'écoulement de liquide.

5. Produit de détection de protéase selon la revendication 3 ou 4, comprenant en outre une zone de réception d'échantillon pour le mélange du composant clivable et de l'échantillon, où le premier emplacement se situe dans la zone de réception d'étanchéité, et où la barrière dégradable ou apte à être rompue se situe en aval de la zone de réception d'échantillon et en amont du composant de capture.

6. Produit de détection de protéase selon l'une quelconque des revendications précédentes, dans lequel le composant de capture comprend une moitié d'une paire de liaisons.

7. Produit de détection de protéase selon la revendication 6, dans lequel le composant de capture comprend un anticorps ou un fragment de liaison d'antigène de celui-ci.

8. Produit de détection de protéase selon la revendication 6, dans lequel le composant de capture comprend la cellulose, l'élément libérable comprenant en outre un fragment de liaison apte à être lié par la cellulose.

9. Produit de détection de protéase selon la revendication 8, dans lequel le fragment de liaison est le galactomannane ou xyloglucane.

10. Produit de détection de protéase selon la revendication 6, dans lequel le composant de capture comprend la biotine ou la streptavidine.

11. Produit de détection de protéase selon l'une quelconque des revendications précédentes, dans lequel le marqueur est une particule d'or ou un groupe fluorophore.

12. Méthode de détection d'une enzyme de protéase dans un échantillon en utilisant un produit de détection de protéase comprenant l'une quelconque des revendications précédentes, comprenant les étapes de: réaliser l'échantillon à l'extrémité amont du chemin d'écoulement de liquide et permettre à l'échantillon de passer le long du chemin d'écoulement de liquide; et déterminer la présence du marqueur au composant de capture et/ou au premier emplacement.

13. Méthode selon la revendication 12, comprenant en outre l'étape consistant à ajouter séparément le marqueur à l'extrémité amont du chemin d'écoulement de liquide et lui permettre de passer le long du chemin d'écoulement de liquide.

14. Méthode selon la revendication 12 ou 13, comprenant en outre l'étape consistant à appliquer un tampon de lavage à l'extrémité amont du chemin d'écoulement de liquide après l'étape consistant à appliquer l'échantillon au chemin d'écoulement de liquide.
